# EUROPÄISCHE PATENTANMELDUNG

(11) **EP 1 157 983 A1**
(43) Veröffentlichungstag der Anmeldung: **28.11.2001**
(21) Anmeldenummer: 01109807.6
(22) Anmeldetag: 21.04.2001
(51) Int. Cl.: C07C 209/60, C07C 211/27, C07D 295/02

(54) **Verfahren zur Herstellung von Arylethylaminen durch Aminierung von Arylolefinen**

(30) Priorität: 20.05.2000 DE 10025114
(71) Anmelder: Aventis Research & Technologies GmbH & Co KG, 65926 Frankfurt am Main (DE)
(72) Erfinder: Trauthwein, Harald, Dr., 80636 München (DE); Beller, Matthias, Prof. Dr., 18119 Rostock (DE); Breindl, Claudia, Dr., 80333 München (DE); Hartung, Christian, 80993 München (DE); Tillack, Annegret, Dr., 18146 Rostock (DE)
(74) Vertreter: Ackermann, Joachim, Dr.

(57) **Zusammenfassung**

Die Erfindung betrifft ein Verfahren zur Herstellung von 2-Arylethylaminderivaten mit einem Alkylsubstituenten in 1-Stellung der Formel (I),

Aryl-CHR¹-CR²(NR³R⁴)-(CHR⁵R⁶)ₙ-CHR⁷R⁸ (I)

durch Umsetzung von aromatischen Olefinen der allgemeinen Formeln (IIa-c)

Aryl-CHR¹-CHR²-(CR⁵R⁶)ₐ-CR⁵=CR⁶-(CR⁵R⁶)_{b}-CHR⁷R⁸ (IIa)

Aryl-CHR¹-CR²=CR⁵-(CR⁵R⁶)_{c}-CHR⁷R⁸ (IIb)

Aryl-CHR¹-CHR²-(CR⁵CR⁶)_{c}-CR⁵=CR⁷CR⁸ (IIc)

mit Aminen der allgemeinen Formel (III),

R³R⁴NH (III)

in Gegenwart einer Base.

## Beschreibung

Die vorliegende Erfindung betrifft ein Verfahren zur Herstellung von 2-Arylethylaminen aus Arylolefinen und Aminen in Gegenwart einer Base als Katalysator.

2-Arylethylaminderivate mit einem Alkylsubstituenten in 1-Stellung (Aryl-CHR¹-CHR²-NR³R⁴, Amphetamine;) sind eine wichtige Verbindungsklasse im Bereich der pharmazeutischen Chemie. In Abhängigkeit von Substituenten weisen 2-Arylethylamine verschiedene biologische Wirkungen auf und haben technische Bedeutung als Pharmazeutika in mehreren Indikationsgebieten. Beispiele für pharmazeutisch eingesetzte Amphetamine sind Fenfluramin (Appetitzügler), Prolintan (Sympathomimetikum), Fenetyllin (Sympathomimetikum) und Bufotenin (Psychodysleptikum).

Generell werden 2-Arylethylamine und ihre Derivate durch Reaktion (nukleophile Substitution) von 2-Arylethylhalogeniden mit Aminen hergestellt. Bei diesem Verfahren werden mindestens stöchiometrische Mengen von Nebenprodukten (Salzabfälle) gebildet. Zusätzlich sind die Ausbeuten der entsprechenden nukleophilen Substitutionen nicht gut, da Mehrfachalkylierungen auftreten. Eine weitere Synthese von 2-Arylethylaminderivaten geht von Arylacetaldehydderivaten aus, die in Gegenwart eines Übergangsmetallkatalysators reduktiv aminiert werden (siehe beispielsweise (a) Glennon, R. A.; Smith, J. D.; Ismaiel, A. M.; EI-Ashmawy, M.; Battaglia, G.; Fisher, J. B. *J*. *Med*. *Chem.* **1991**, *34,* 1094; (b) Nicols, D. E. *J. Med. Chem.* **1973**, *16*, 480). Probleme dieses Verfahrens sind die Zugänglichkeit der Arylacetaldehydderivate und die Kosten des Übergangsmetallkatalysators. Ein Verfahren, das die Nachteile der oben genannten Laborsynthesen vermeidet, ist die basenkatalysierte Aminierung von Styrolen. Hier werden atomeffizient Amine an Styrole in Gegenwart einer Base addiert. Beispiele für diese Reaktion findet man in Beller, M.; Breindl, C. *Tetrahedron* **1998,** *54*, 6359. Problematisch wird dieses Verfahren jedoch, wenn Amphetamine, d.h. 2-Arylethylamine mit einem weiteren Alkylsubstituenten in 1-Stellung, synthetisiert werden sollen, da die notwendigen Ausgangsverbindungen nicht einfach und praktikabel darstellbar sind.

Aus den genannten Gründen bestand ein Bedarf nach einem neuen Verfahren, das 2-Arylethylaminderivate mit einem Alkylsubstituenten in 1-Stellung einfach, aus kostengünstigen, leicht erhältlichen Edukten zugänglich macht, und das nicht die Nachteile der bekannten Herstellverfahren zeigt und somit für eine technische Durchführung geeignet ist und das 2-Arylethylaminderivate mit einem Alkylsubstituenten in 1-Stellung in hoher Ausbeute, Katalysatorproduktivität und Reinheit liefert.

Es konnte überraschend festgestellt werden das in Gegenwart von Basen 2-Arylethylaminderivate mit einem Alkylsubstituenten in 1-Stellung aus Arylolefinen der Formeln (lla-c) und Aminen erhältlich sind. Dabei erfolgt die Aminierung mit hoher Selektivität in 2-Stellung des Arylolefins, nahezu unabhängig von der Lage der Doppelbindung im olefinischen Rest.

Somit werden die leichter zugänglichen Arylolefine, die eine Doppelbindung in 2-Stellung oder höher im olefinischen Rest besitzen, für die Herstellung von 2-Arylethylaminderivate mit einem Alkylsubstituenten in 1-Stellung verwendbar. Der gezielten Aminierung in 2-Stellung des Arylolefins scheint eine lsomerisierungsreaktion (Domino-lsomerisierung) vorauszugehen. Damit können bei der Synthese eines bestimmten 2-Arylethylaminderivates auch direkt Isomerengemische eines Arylolefins mit unterschiedlicher Stellung der Doppelbindung eingesetzt werden.

Gegenstand der Erfindung ist folglich ein Verfahren zur Herstellung von 2-Arylethylaminderivaten der Formel (I),

Aryl-CHR¹-CR²(NR³R⁴)-(CHR⁵R⁶)ₙ-CHR⁷R⁸ (I)

worin R¹ bis R⁶
gleich oder verschieden Wasserstoff, C₁-C₂₄-Alkyl, C₃-C₁₂-Cycloalkyl, wobei der Cyclus auch 1-2 Heteroatome ausgewählt aus der Gruppe N,O, S, P enthalten kann und die Ringgröße 3-12 beträgt, Phenyl, Naphthyl, Fluorenyl, C₆-C₁₄-Aryl, C₂-C₁₃-Heteroaryl, wobei die Zahl der Heteroatome ausgewählt aus der Gruppe N,O, S, P 1 bis 4 betragen kann und die Ringgröße 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoralkylalkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, C₁-C₉-Trifluoromethylalkyl, Trifluormethyl, Fluoro, Nitro, Hydroxy, Trifluormethylsulfonato, Thio, Thiolato, C₁-C₈-substituierte Amino der Formen, NH-Alkyl-C₁-C₈, NH-Aryl-C₅-C₆, N-Alkyl₂-C₁-C₈, N-Aryl₂-C₅-C₆, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, C₁-C₇-CO₂H, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃ R⁹₂, CONH₂, CONR⁹₂, CONHR⁹, wobei R⁹ entweder ein C₁-C₈-Alkyl oder C₆-Aryl darstellt, C₁-C₆-Trialkylsilyl, sein können,
wobei zur Klarstellung daraufhingewiesen wird, dass auch die einzelnen Substituenten R⁵ bzw. R⁶ in den n Untereinheiten als voneinander unabhängige Substituenten anzusehen sind, und
worin R⁷ und R⁸
gleich oder verschieden Wasserstoff, C₁-C₂₄-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, Naphthyl, Fluorenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, Fluor, OH, NO₂, CN, O-C₁-C₂₄-Alkyl, O-C₃-C₁₂-Cycloalkyl, wobei der Cyclus auch 1-2 Heteroatome ausgewählt aus der Gruppe N, O, S, P enthalten kann und die Ringgröße 3-12 beträgt, O-C₆-C₁₄-Aryl, O-C₂-C₁₃-Heteroaryl, wobei die Zahl der Heteroatome ausgewählt aus der Gruppe N, O, S, P 1 bis 4 betragen kann und die Ringgröße 3-14 beträgt, N-(C₁-C₂₄)₂-Alkyl, N-(C₃-C₈)₂-Cycloalkyl, wobei der Cyclus auch 1-2 Heteroatome ausgewählt aus der Gruppe N, O, S, P enthalten kann und die Ringgröße 3-12 beträgt,
N-(C₆-C₁₄-Aryl)₂, N-(C₂-C₁₃-Heteroaryl)₂, wobei die Zahl der Heteroatome ausgewählt aus der Gruppe N, O, S, P 1 bis 4 betragen kann und die Ringgröße 3-14 beträgt,
O₂C-C₁-C₂₄-Alkyl, O₂C-C₃-C₈-Cycloalkyl wobei der Cyclus auch 1-2 Heteroatome ausgewählt aus der Gruppe N, O, S, P enthalten kann und die Ringgröße 3-12 beträgt, O₂C-C₆-C₁₄-Aryl,
NR¹⁰-CO-(C₁-C₂₄)₂-Alkyl, NR¹⁰-CO-(C₃-C₁₂)₂-Cycloalkyl, wobei der Cyclus auch 1-2 Heteroatome ausgewählt aus der Gruppe N, O, S, P enthalten kann und die Ringgröße 3-12 beträgt,
NR¹⁰-CO-(C₆-C₁₄-Aryl)₂, NR¹⁰-CO-(C₂-C₁₃-Heteroaryl)₂, wobei die Zahl der Heteroatome ausgewählt aus der Gruppe N, O, S, P 1 bis 4 betragen kann und die Ringgröße 3-14 beträgt, wobei R¹⁰ C₁-C₈-Alkyl oder C₆-Aryl darstellt
Si-(C₁-C₂₄)₄-Alkyl, Si-(C₃-C₁₂)₄-Cycloalkyl, wobei der Cyclus auch 1-2 Heteroatome ausgewählt aus der Gruppe N, O, S, P enthalten kann und die Ringgröße 3-12 beträgt,
Si-(C₆-C₁₄-Aryl)₄, Si-(C₂-C₁₃-Heteroaryl)₄, wobei die Zahl der Heteroatome ausgewählt aus der Gruppe N, O, S, P 1 bis 4 betragen kann und die Ringgröße bevorzugt 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoralkyl, C₃-C₁₂-Cycloalkyl, wobei der Cyclus auch 1-2 Heteroatome ausgewählt aus der Gruppe N,O,S,P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy; C₃-C₁₃-Heteroaryloxy, wobei die Zahl der Heteroatome ausgewählt aus der Gruppe N,O,S,P 1 bis 4 betragen kann und die Ringgröße 3-14 beträgt, C₁-C₉-Trifluormethylalkyl, Trifluormethyl, Fluoro, Nitro, Hydroxy, Trifluormethylsulfonato, Thio, Thiolato, C₁-C₈-substituierte Amino der Formen, NH-Alkyl-C₁-C₈, NH-Aryl-C₅-C₆, N-Alkyl₂-C₁-C₈, N-Aryl₂-C₅-C₆, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, C₁-C₇-CO₂H, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃ R⁹₂, CONH₂, CONR⁹₂, CONHR⁹, wobei R⁹ entweder ein C₁-C₈-Alkyl oder C₆-Aryl darstellt, C₁-C₆-Trialkylsilyl, sein können,
und worin Aryl in Formel (I)
C₃-C₁₄-Aryl, C₂-C₁₃-Heteroaryl, wobei die Zahl der Heteroatome ausgewählt aus der Gruppe N,O,S,P 1 bis 4 betragten kann und die Ringgröße 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoroalkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₁-C₉-Trifluoromethylalkyl, Trifluormethyl, Fluoro, Nitro, Hydroxy, Trifluormethylsulfonato, Thio, Thiolato, C₁-C₈-substituierte Amino der Formen NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, CHCHCO₂H, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃R⁹₂, CONH₂, CONR⁹₂, CONHR⁹, wobei R⁹ entweder C₁-C₈-Alkyl oder C₆-Aryl darstellt, C₁-C₆-Trialkylsilyl, sein können, und
worin n für eine Zahl zwischen 0 und 11 steht,
durch Umsetzung von aromatischen Olefinen der allgemeinen Formeln (lla-c)

Aryl-CHR¹-CHR²-(CR⁵R⁶)ₐ-CR⁵=CR⁶-(CR⁵R⁶)_{b}-CHR⁷R⁸ (IIa)

Aryl-CHR¹-CR²=CR⁵-(CR⁵R⁶)_{c}-CHR⁷R⁸ (IIb)

Aryl-CHR¹-CHR²-(CR⁵CR⁶)_{c}-CR⁵=CR⁷CR⁸ (IIc)

mit Aminen der allgemeinen Formel (III),

R³R⁴NH (III)

worin bei den Formeln des Typs II und III R¹ bis R⁸ die zuvor zu den Formeln des Typs (I) angegebene Bedeutung besitzen und a sowie b für eine Zahl zwischen 0 - 9 mit der Bedingung, daß a + b < 10 ist und c für eine Zahl zwischen 0 - 10 steht,
in Gegenwart einer Base, insbesondere einer Brönsted-Base. Bevorzugte Basen sind Alkali- und/oder Erdalkalimetalle (z.B. Natrium, Lithium, Kalium, Calcium), Alkali- und/oder Erdalkalihydride (z.B. Natriumhydrid, Lithiumhydrid, Magnesiumhydrid, Calciumhydrid), Alkali- und/oder Erdalkaliamide (z.B. Lithiumdiisopropylamid, Natriumamid, Lithiumdiethylamid, Natriumdimethylamid) Alkali- und/oder Erdalkalialkoholate (z.B. Kaliumtertbutylat) sowie Alkali- und/oder Erdalkalikohlenwasserstoffe (z.B. Butyllithium, Methyllithium, Phenyllithium, Phenylnatrium, Diethylmagnesium). Besonders bevorzugt sind Alkalimetalle, Alkali- und/oder Erdalkalihydride, Alkaliamide sowie Alkalikohlenwasserstoffe.

Besonders bevorzugt ist ein Verfahren, bei dem Verbindungen der Formel (I) hergestellt werden, worin R¹ bis R⁶
für Wasserstoff, C₁-C₈-Alkyl, C₆-C₁₄-Aryl, C₃-C₁₂-Cycloalkyl, wobei der Cyclus auch 1-2 Heteroatome ausgewählt aus der Gruppe N, O, S, P enthalten kann, stehen und die Ringgröße 3-12 beträgt,
wobei diese Gruppen selbst jeweils ein- bis dreifach substituiert sein können und diese Substituenten dabei unabhängig voneinander Wasserstoff, C₁-C₂₀-Alkyl, C₆-C₁₄-Aryl, C₃-C₁₂-Cycloalkyl, wobei der Cyclus auch 1-2 Heteroatome ausgewählt aus der Gruppe N, O, S, P enthalten kann und die Ringgröße 3-12 beträgt, C₁-C₁₀-Alkoxy, C₁-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, Fluor, Trifluormethyl, N-Alkyl₂-C₁-C₈, N-Aryl₂-C₅-C₆ bedeuten.
und worin R⁷ und R⁸ unabhängig voneinander Wasserstoff, Fluor, Trifluormethyl, C₆-C₁₄-Aryl, O-C₁-C₈-Alkyl, O-C₃-C₁₂-Cycloalkyl, wobei der Cyclus auch 1-2 Heteroatome ausgewählt aus der Gruppe N, O, S, P enthalten kann und die Ringgröße 3-12 beträgt, O-C₆-C₁₄-Aryl, O-C₂-C₁₃-Heteroaryl, wobei die Zahl der Heteroatome ausgewählt aus der Gruppe N, O, S, P 1 bis 4 betragen kann und die Ringgröße bevorzugt 3-14 beträgt,
N-(C₁-C₈)₂-Alkyl, N-(C₃-C₈)₂-Cycloalkyl, wobei der Cyclus auch 1-2 Heteroatome ausgewählt aus der Gruppe N, O, S, P enthalten kann und die Ringgröße 3-12 beträgt,
N-(C₆-C₁₄-Aryl)₂, N-(C₂-C₁₃-Heteroaryl)₂, wobei die Zahl der Heteroatome ausgewählt aus der Gruppe N, O, S, P 1 bis 4 betragen kann und die Ringgröße 3-14 beträgt,
wobei diese Gruppen selbst jeweils ein- bis dreifach substituiert sein können und diese Substituenten dabei unabhängig voneinander Wasserstoff, C₁-C₂₀-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, N-Alkyl₂-C₁-C₈, N-Aryl₂-C₅-C₆, Trifluormethyl, Fluoro sein können.
Besonders bevorzugte Substituenten sind auch Phenyl, Naphthalin, Phenanthren, Pyrrol, Furan, Thiophen, Indol, Chinolin, Benzofuran
und worin Aryl in Formel (I)
für C₃-C₁₄-Aryl, C₂-C₁₃-Heteroaryl, wobei die Zahl der Heteroatome ausgewählt aus der Gruppe N, O, S, P 1 bis 4 betragten kann und die Ringgröße 3-14 beträgt,
wobei diese Gruppen selbst jeweils ein- bis dreifach substituiert sein können und diese Substituenten dabei unabhängig voneinander Wasserstoff, C₁-C₂₀-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₁-C₉-Trifluoromethylalkyl, Trifluormethyl, Fluoro, Nitro, Hydroxy, Trifluormethylsulfonato, Thio, Thiolato
und worin n für eine Zahl zwischen 0 und 7 steht.

Die bevorzugten Ringgrößen der Cycloalkyl-, Heterocycloalkyl-, Aryl und Heteroarylsubstituenten liegt bei 5 bis 7.

Das erfindungsgemäße Verfahren hat sich ganz besonders für die Herstellung von Amphetaminen bewährt, worin R¹ bis R⁶ unabhängig voneinander für Wasserstoff, C₁-C₈-Alkyl, C₆-C₁₄-Aryl, C₃-C₁₂-Cycloalkyl, wobei der Cyclus auch 1-2 Heteroatome ausgewählt aus der Gruppe N, O, S, P enthalten kann und die Ringgröße 3-12 beträgt,
und worin R⁷ und R⁸ Wasserstoff C₃-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, bedeuten,
und worin n für eine Zahl zwischen 0 und 7 steht.

Im Rahmen des erfindungsgemäßen Verfahrens können auch vorteilhaft Olefinmischungen der Formel II eingesetzt werden, wobei man selektiv Arylethylamine der Formel I als Produkte erhält. Olefinmischungen der Formel II können beispielsweise durch Heck-Reaktion von Arylhalogeniden mit kostengünstigen Olefinen hergestellt werden (siehe M. Beller, T. H. Riermeier, G.

Stark in Transition Metals for Organic Synthesis (Eds. M. Beller, C. Bolm) Vol. I, S. 208 - 240, Wiley-VCH, Weinheim, 1998).

Als Lösungsmittel des Verfahrens finden im allgemeinen inerte organische Lösungsmittel Verwendung. Besonders geeignet sind aliphatische und aromatische Ether (MTBE, THF, Dioxan, Anisol, Diethylether, Dibutylether, etc.), Polyether (Polyethylenglykole, etc.), aromatische und/oder aliphatische Kohlenwasserstoffe (Toluol, Xylol, Tetralin, Octan, etc.) sowie deren Gemische. Daneben kann die Reaktion auch in tert. Aminen (Triethylamin, Tributylamin, Methyldiisopropylamin, etc.), dipolar aprotischen Lösemitteln (DMSO, DMAc, NMP, Tetramethylharnstoff, etc.) oder in Substanz durchgeführt werden.

Die Reaktion läuft bei Temperaturen von -70 bis 200 °C ab; in vielen Fällen hat es sich bewährt, bei Temperaturen von 0 bis 180 °C, bevorzugt 20 bis 140 °C, zu arbeiten. Die Reaktion kann unter Druck durchgeführt werden, insbesondere wenn niedrig siedende Amine eingesetzt werden.

Als Katalysator muß dem Reaktionsgemisch eine Base zugesetzt werden. Der basische Katalysator dient dazu, das vorhandene Amin zum entsprechenden Amid zu deprotonieren. Sowohl die Base als auch das entsprechende Amid katalysieren die Isomerisierung der Doppelbindung und die nachfolgende Aminierung. Als Basen sind solche Verbindungen geeignet, die das Amin in geringer Konzentration zu deprotonieren vermögen. Dafür besonders geeignet sind Alkali- oder/und Erdalkalialkoholate wie z.B. KOtBu, Alkali- oder/und Erdalkaliamide, Alkali- oder/und Erdalkalielemente oder/und Alkali- oder/und Erdalkalikohlenwasserstoffe wie z.B. Butyllithium, Phenyllithium oder/und Hydroxide bevorzugt von Lithium, Natrium, Kalium, Calcium, Magnesium, Cäsium. Es können auch sogenannte feste Superbasen (z.B. alkalimetalldotierte Zeolithe) als Katalysatoren eingesetzt werden.

Bei dem erfindungsgemäßen Verfahren wird die Base bevorzugt in katalytischen Mengen bezogen auf das Amin eingesetzt. Bevorzugt sind Katalysatormengen von 0.01 eq bis 0.5 eq verwendet. Besonders bevorzugt werden 0.05 eq - 0.4 eq Basenkatalysator eingesetzt.

In einigen Fällen hat es sich als positiv herausgestellt, einen das intermediär entstehende Alkali- bzw. Erdalkaliamid stabilisierenden Co-Katalysator zuzusetzen. Als Co-Katalysatoren können insbesondere chelatisierende Diamine wie TMEDA aber auch Trialkylamine oder Ether, die alicyclisch oder/und offenkettig sein können, eingesetzt werden. Der Co-Katalysator wird in Mengen von 0.01 eq bis 1.5 eq (bezogen auf Amin) verwendet. Bevorzugt werden 0.05 eq - 1 eq Co-Katalysator eingesetzt.

Die erfindungsgemäß herstellbaren Amine sind als Pharmazeutika von großer Bedeutung.

### Beispiele:

Die nachstehenden Beispiele dienen zur Erläuterung des erfindungsmäßigen Verfahrens, ohne es darauf zu beschränken.

### Allgemeine Arbeitsvorschrift:

Das Amin (2.5 mmol) und 100 µl Hexadecan (interner GC-Standard) werden unter Argon-Schutzgasatmosphäre in 5 ml absolutem Tetrahydrofuran in einem Aldrich Ace-Druckrohr gelöst. 20 mol% *n*-Butyllithium (1.6 M *n*-BuLi-Lösung in Hexan) werden langsam bei Raumtemperatur zugegeben. Die Lösung wird für 10 Minuten gerührt, bevor Allylbenzol (5 mmol) zugegeben wird. Die zumeist intensiv gefärbte Lösung wird für 20 Stunden bei der angegebenen Temperatur umgesetzt. Nach dem Abkühlen wird die Mischung mit 2 ml Wasser hydrolysiert, wobei Entfärbung der Lösung auftritt. Zur Isolierung des Produkts wird der Ansatz mit 5 ml 1 M Salzsäure und 5 ml Dichlormethan versetzt. Die wäßrige Phase wird abgetrennt und die organische Phase dreimal mit jeweils 5 ml 1 M Salzsäure extrahiert. Die vereinigten wäßrigen Phasen werden mit Na₂CO₃ neutralisiert und fünfmal mit jeweils 5 ml Dichlormethan extrahiert. Die organischen Phasen werden mit Wasser gewaschen und über MgSO₄ getrocknet. Nach Entfernen des Lösungsmittels im Vakuum wird das Produkt mittels Säulenchromatographie isoliert.

### Beispiel 1:

***N-2-(1-Phenyl)propyl-piperidin.*** Piperidin (2.5 mmol; 247 µl) und Allylbenzol (5 mmol; 662 µl) werden gemäß der allgemeinen Arbeitsvorschrift unter Zusatz von 20 mol% *n*-BuLi-Lösung (0.5 mmol; 313 µl) bei Raumtemperatur umgesetzt. Die Reinigung des Produkts erfolgt säulenchromatographisch (*n*-Hexan/Ethylacetat = 3:1). N-2-(1-Phenyl)propyl-piperidin wird als farbloses Öl erhalten. - Ausbeute: 89 % (GC); 84 % (isoliert). - ¹H NMR (CDCl₃, 400.1 MHz, 25 °C, δ = ppm): 7.24 (m, 2H, Phenyl); 7.15 (m, 3H, Phenyl); 3.00 (dd, ²*J*(H, H) = 12.9 Hz, ³*J*(H, H) = 3.8 Hz, 1H, Ph-C*H*₂); 2.76 (ddq, ³*J*(H, H) = 10.1 Hz, ³*J*(H, H) = 6.5 Hz, ³*J*(H, H) = 3.8 Hz, 1H, Ph-CH₂-C*H*); 2.54 (m, 4H, N-C*H*₂); 2.36 (dd, ²*J*(H, H) = 12.9 Hz, ³*J*(H, H) = 10.1 Hz, 1H, Ph-C*H*₂); 1.59 (m, 4H, N-CH₂-C*H*₂); 1.43 (m, 2H, N-CH₂-CH₂-C*H*₂); 0.91 (d, ³*J*(H, H) = 6.5 Hz, 3H, C*H*₃). ¹³C NMR (CDCl₃, 100.6 MHz, 25 °C, δ = ppm): 141.0, 129.2, 128.1, 125.7, 62.1, 49.6, 39.1, 26.4, 24.9, 14.2. GC-MS: *m/z* = 203 [M⁺], 112 [M⁺-C₆H₅-CH₂], 91, 69. MS (Cl, Isobutan): 204 [M⁺ + H], 112 [M⁺ - C₆H₅-CH₂], 86. *Anal.* ber. für C₁₄H₂₁N: C 82.70, H 10.41, N 6.89. gef.: C 82.68, H 10.42, N 6.85.

### Beispiel 2:

***N-2-(1-Phenyl)propyl-morpholin*.** Morpholin (2.5 mmol; 218 µl) und Allylbenzol (5 mmol; 662 µl) werden gemäß der allgemeinen Arbeitsvorschrift unter Zusatz von 20 mol% n-BuLi-Lösung (0.5 mmol; 313 µl) bei 50 °C umgesetzt. Die Reinigung des Produkts erfolgt säulenchromatographisch (*n*-Hexan/Ethylacetat = 1:1). N-2-(1-Phenyl)propylmorpholin wird als farbloses Öl erhalten. - Ausbeute: 88 % (GC); 80 % (isoliert). - ¹H NMR (CDCl₃, 400.1 MHz, 25 °C, δ = ppm): 7.26 (m, 2H, Phenyl); 7.16 (m, 3H, Phenyl); 3.72 (m, 4H, O-C*H*₂); 2.99 (dd, ²*J*(H, H) = 13.1 Hz, ³*J*(H, H) = 4.4 Hz, 1H, Ph-C*H*₂); 2.75 (ddq, ³*J*(H, H) = 9.7 Hz, ³*J*(H, H) = 6.5 Hz, ³*J*(H, H) = 4.4 Hz, 1H, Ph-CH₂-C*H*); 2.60 (m, 4H, N-C*H*₂); 2.39 (dd, ²*J*(H, H) = 13.1 Hz, ³*J*(H, H) = 9.7 Hz, 1H, Ph-C*H*₂); 0.94 (d, ³*J*(H, H) = 6.5 Hz, 3H, C*H*₃). ¹³C NMR (CDCl₃, 100.6 MHz, 25 °C, δ = ppm): 140.3, 129.2, 128.2, 125.8, 67.3, 61.6, 49.0, 39.1, 14.2. GC-MS: *m/z =* 114 [M⁺ - C₆H₅-CH₂], 91, 70. MS (Cl, Isobutan): 206 [M⁺ + H], 114 [M⁺ - C₆H₅-CH₂]. *Anal.* ber. für C₁₃H₁₉NO: C 76.06, H 9.33, N 6.82. gef.: C 76.06, H 9.19, N 6.88.

### Beispiel 3:

***N-2-(1-Phenyl)propyl-N-benzylamin*** Benzylamin (2.5 mmol; 273 µl) und Allylbenzol (5 mmol; 662 µl) werden gemäß der allgemeinen Arbeitsvorschrift unter Zusatz von 20 mol% *n*-BuLi-Lösung (0.5 mmol; 313 µl) bei 50 °C umgesetzt. Die Reinigung des Produkts erfolgt säulenchromatographisch (*n*-Hexan/Ethylacetat = 4:1). *N*-2-(1-Phenyl)propyl-*N*-benzylamin wird als farbloses Öl erhalten. - Ausbeute: 65 % (GC); 60 % (isoliert). - ¹H NMR (CDCl₃, 400.1 MHz, 25 °C, δ = ppm): 7.29 - 7.12 (m, 10H, Phenyl); 3.83 (d, ²*J*(H, H) = 13.3 Hz, 1H, Ph-C*H*₂-N); 3.72 (d, ²*J*(H, H) = 13.3 Hz, 1H, Ph-C*H*₂-N); 2.92 (sext, 1H, ³*J*(H, H) = ^{*3*}*J*(H, H) = 6.3 Hz, 1H, Ph-CH₂-C*H*); 2.76 (dd, ²*J*(H, H) = 13.3 Hz, ³*J*(H, H) = 6.3 Hz, 1H, Ph-C*H*₂-CH); 2.62 (dd, ²*J*(H, H) = 13.3 Hz, ³*J*(H, H) = 6.3 Hz, 1 H, Ph-C*H*₂-CH); 1.54 (s, 1H, N*H*); 1.08 (d, ³*J*(H, H) = 6.3 Hz, 3H, C*H*₃). ¹³C NMR (CDCl₃, 100.6 MHz, 25 °C, δ = ppm): 140.4, 139.4, 129.3, 128.3, 128.3, 127.9, 126.8, 126.1, 53.7, 51.2, 43.5, 20.1. GC-MS: *m/z* = 134 [M⁺ - C₆H₅-CH₂], 91, 65. MS (Cl, Isobutan): 226 [M⁺ + H], 134 [M⁺ - C₆H₅-CH₂]. *Anal*. ber. für C₁₆H₁₉N: C 85.28, H 8.50, N 6.22. gef.: C 85.19, H 8.51, N 6.19.

### Beispiel 4:

***N-(S)-1-Phenylethyl-N-2-(1-phenyl)propyl- amin*** *S*-(-)-α-Methylbenzylamin (2.5 mmol; 318 µl) und Allylbenzol (5 mmol; 662 µl) werden gemäß der allgemeinen Arbeitsvorschrift unter Zusatz von 20 mol% *n*-BuLi-Lösung (0.5 mmol; 313 µl) und 20 mol% Tetramethylethylendiamin (0.5 mmol, 75 µl) bei 50 °C umgesetzt. Die Reinigung des Produkts erfolgt säulenchromatographisch (*n*-Hexan/Ethylacetat = 2:1). *N*-*(S)*-1-Phenylethyl-*N*-2-(1-phenyl)propyl-amin wird als farbloses Öl erhalten.-Ausbeute: 32 % (GC); 30 % (isoliert). ¹H NMR (CDCl₃, 400.1 MHz, 25 °C, δ = ppm): 7.36 - 6.94 (m, 2· 10H, Phenyl); 3.93, 3.88 (2· q, ³*J*(H, H) = 6.6 Hz, 2· 1 H, Ph-C*H*Me-N); 2.88, 2.66 (2· dd, ²*J*(H, H) = 12.9 Hz, ³*J*(H, H) = 5.0 Hz, 2· 1 H, Ph-C*H*₂); 2.77, 2.65 (2· m, 2· 1H, Ph-CH₂-C*H*); 2.59, 2.50 (2· dd, ²*J*(H, H) = 12.9 Hz, ³*J*(H, H) = 7.5 Hz, 2· 1 H, Ph-C*H*₂); 1.46 (2· s, 2· 1 H, N*H*); 1.31, 1.27 (2· d, ³*J*(H, H) = 6.7 Hz, 2· 3H, Ph-CH(C*H*₃)-N); 1.05, 0.92 (2· d, ³*J*(H, H) = 6.3 Hz, 2· 3H, Ph-CH₂-CH-C*H*₃). ¹³C NMR (CDCl₃, 100.6 MHz, 25 °C, δ = ppm): 146.1, 145.4, 139.5, 139.3, 129.4, 129.2, 128.4, 128.3, 128.3, 128.2, 126.8, 126.6, 126.5, 126.3, 126.1, 125.9, 55.3, 54.8, 51.9, 50.8, 44.2, 42.5, 25.0, 24.5, 21.1, 19.9. GC-MS: *m/z* = 239 [M⁺], 148 [M⁺-C₆H₅-CH₂], 105 [C₆H₅-OH-CH₃], 91, 79. *Anal*. ber. für C₁₇H₂₁N: C 85.30, H 8.84, N 5.85. gef.: C 85.20, H 8.88, N 5.86.

### Beispiel 5:

***N-n-Butyl-N-2-(1-phenyl)propyl-amin.*** *n*-Butylamin (2.5 mmol; 247 µl) und Allylbenzol (5 mmol; 662 µl) werden gemäß der allgemeinen Arbeitsvorschrift unter Zusatz von 20 mol% *n*-BuLi-Lösung (0.5 mmol; 313 µl) bei 50 °C umgesetzt. Die Reinigung des Produkts erfolgt säulenchromatographisch (*n*-Hexan/Ethylacetat = 1:2). *N-n*-Butyl-*N*-2-(1-phenyl)propyl-amin wird als farbloses Öl erhalten. - Ausbeute: 62 % (GC); 54 % (isoliert). - ¹H NMR (CDCl₃, 400.1 MHz, 25 °C, δ = ppm): 7.27 (m, 2H, Phenyl); 7.17 (m, 3H, Phenyl); 2.87 (sext, ³*J*(H, H) = ³*J*(H, H) = 6.5 Hz, 1H, Ph-CH₂-C*H*); 2.73 (dd, ²*J*(H, H) = 13.3 Hz, ³*J*(H, H) = 6.5 Hz, 1H, Ph-C*H*₂); 2.65 (ddd, ²*J*(H, H) = 11.1 Hz, ³*J*(H, H) = 8.1 Hz, ³*J*(H, H) = 6.5 Hz, 1H, N-C*H*₂); 2.58 (dd, ²*J*(H, H) = 13.3 Hz, ³*J*(H, H) = 6.5 Hz, 1H, Ph-C*H*₂); 2.51 (ddd, ²*J*(H, H) = 11.1 Hz, ³*J*(H, H) = 7.6 Hz, ³*J*(H, H) = 6.7 Hz, 1H, N-C*H*₂); 1.40 (m, 2H, N-CH₂-C*H*₂); 1.37 (s, 1H, N*H*); 1.25 (sext, ³*J*(H, H) = ³*J*(H, H) = 7.4 Hz, 2H, CH₂-C*H*₂-CH₃); 1.04 (d, ³*J*(H, H) = 6.5 Hz, 3H, CH-C*H*₃); 0.85 (t, ³*J*(H, H) = 7.4 Hz, 3H, CH₂-C*H*₃). ¹³C NMR (CDCl₃, 100.6 MHz, 25 °C, δ = ppm): 139.5, 129.2, 128.3, 126.1, 54.7, 47.0, 43.6, 32.3, 20.4, 20.2, 13.9. GC-MS: *m/z* = 191 [M⁺], 176 [M⁺ - CH₃], 100 [M⁺ - C₆H₅-CH₂], 91, 77. *Anal.* ber. für C₁₃H₂₁N: C 81.61, H 11.06, N 7.32. gef.: C 81.44, H 10.99, N 7.20.

### Beispiel 6:

***N-n-Butyl-N-methyl-N-2-(1-phenyl)propyl-amin*.** *n*-Butylmethylamin (2.5 mmol; 296 µl) und Allylbenzol (5 mmol; 662 µl) werden gemäß der allgemeinen Arbeitsvorschrift unter Zusatz von 20 mol% *n*-BuLi-Lösung (0.5 mmol; 313 µl) bei Raumtemperatur umgesetzt. Die Reinigung des Produkts erfolgt säulenchromatographisch (*n*-Hexan/Ethylacetat = 3:1). *N*-*n*-Butyl-*N*-methyl-*N*-2-(1-phenyl)propyl-amin wird als farbloses Öl erhalten. - Ausbeute: 66 % (GC); 61 % (isoliert). - ¹H NMR (CDCl₃, 400.1 MHz, 25 °C, δ = ppm): 7.26 (m, 2H, Phenyl); 7.17 (m, 3H, Phenyl); 2.94 (dd, ²*J*(H, H) = 12.5 Hz, ³*J*(H, H) = 4.0 Hz, 1H, Ph-C*H*₂); 2.89 (m, 1H, Ph-CH₂-C*H*); 2.44 (m, 2H, N-C*H*₂); 2.38 (dd, ²*J*(H, H) = 12.5 Hz, ³*J*(H, H) = 9.5 Hz, 1H, Ph-C*H*₂); 2.28 (s, 3H, N-C*H*₃); 1.46 (m, 2H, N-CH₂-C*H*₂); 1.31 (m, 2H, CH₂-C*H*₂-CH₃); 0.91 (t, ³*J*(H, H) = 7.3 Hz, 3H, CH₂-C*H*₃); 0.90 (d, ³*J*(H, H) = 6.9 Hz, 3H, CH-C*H*₃). ¹³C NMR (CDCl₃, 100.6 MHz, 25 °C, δ = ppm): 140.8, 129.2, 128.2, 125.7, 60.3, 53.2, 39.0, 37.1, 30.4, 20.7, 14.1, 13.9. GC-MS: *m/z* = 205 [M⁺], 190 [M⁺ - CH₃], 162 [M⁺ - CH₂CH₂CH₃], 114 [M⁺ - C₆H₅-CH₂], 91, 72, 58. *Anal.* ber. für C₁₄H₂₃N: C 81.89, H 11.29, N 6.82. gef.: C 81.31, H 11.19, N 6.76.

### Beispiel 7:

***N-2-(1-Phenyl)propyl-anilin..*** Anilin (2.5 mmol; 228 µl) und Allylbenzol (2.5 mmol; 331 µl) werden gemäß der allgemeinen Arbeitsvorschrift unter Zusatz von 30 mol% *n*-BuLi-Lösung (0.75 mmol; 469 µl) und 30 mol% KO^{*t*}Bu (0.75 mmol, 84 mg) bei 120 °C umgesetzt. Die Reinigung des Produkts erfolgt säulenchromatographisch (*n*-Hexan/Ethylacetat = 15:1). *N*-2-(1-Phenyl)propyl-anilinwird als farbloses Öl erhalten. - Ausbeute: 54 % (GC); 50 % (isoliert). - ¹H NMR (CDCl₃, 400.1 MHz, 25 °C, δ = ppm): 7.28 (m, 2H, Phenyl); 7.18 (m, 3H, Phenyl); 7.16 (m, 2H, Phenyl); 6.68 (m, 1H, Phenyl); 6.62 (m, 2H, Phenyl); 3.75 (m, 1H, Ph-CH₂-C*H*); 3.61 (s, 1H, N*H*); 2.93 (dd, ²*J*(H, H) = 13.4 Hz, ³*J*(H, H) = 4.8 Hz, 1H, Ph-C*H*₂); 2.68 (dd, ²*J*(H, H) = 13.4 Hz, ³*J*(H, H) = 7.3 Hz, 1H, Ph-C*H*₂); 1.13 (d, ³*J*(H, H) = 6.3 Hz, 3H, C*H*₃). ¹³C NMR (CDCl₃, 100.6 MHz, 25 °C, δ = ppm): 147.1, 138.5, 129.5, 129.3, 128.3, 126.2, 117.2, 113.4, 49.4, 42.2, 20.1. GC-MS: *m/z* = 211 [M⁺], 120 [M⁺ - C₆H₅-CH₂], 91, 77. *Anal.* ber. für C₁₅H₁₇N: C 85.26, H 8.11, N 6.63. gef.: C 85.41, H 8.10, N 6.64.

### Beispiel 8:

***N-2-(1-phenyi)butyi-piperidin.*** Piperidin (2.5 mmol; 247 µl) und 4-Phenyl-1-buten (5 mmol; 746 µl) werden gemäß der allgemeinen Arbeitsvorschrift unter Zusatz von 20 mol% *n*-BuLi-Lösung (0.5 mmol; 313 µl) bei 50 °C umgesetzt. Die Reinigung des Produkts erfolgt säulenchromatographisch (*n*-Hexan/Ethylacetat = 4:1). *N*-2-(1-phenyl)butyl-piperidin. wird als farbloses Öl erhalten. - Ausbeute: 59 % (GC); 57 % (isoliert). - ¹H NMR (CDCl₃, 400.1 MHz, 25 °C, δ = ppm): 7.25 (m, 2H, Phenyl); 7.16 (m, 3H, Phenyl); 2.94 (dd, ²*J*(H, H) = 12.9 Hz, ³*J*(H, H) = 4.0 Hz, 1H, Ph-C*H*₂); 2.66-2.40 (m, 5H, N-C*H*₂, Ph-CH₂-C*H*); 2.33 (dd, ²*J*(H, H) = 12.9 Hz, ³*J*(H, H) = 9.0 Hz, 1H, Ph-C*H*₂); 1.54 (m, 4H, N-CH₂-C*H*₂); 1.49 - 1.38 (m, 3H, N-CH₂-CH₂-C*H*₂, Ph-CH₂-CH-C*H*₂); 1.32 (m, 1H, Ph-CH₂-CH-C*H*₂); 0.81 (t, ³*J*(H, H) = 7.3 Hz, 3H, C*H*₃). ¹³C NMR (CDCl₃, 100.6 MHz, 25 °C, δ = ppm): 141.7, 129.2, 128.1, 125.4, 68.6, 49.6, 35.5, 26.6, 25.1, 23.3, 11.7. GC-MS: *m/z* = 217 [M⁺], 188 [M⁺ - CH₂CH₃], 126 [M⁺ - C₆H₅-CH₂], 91, 69. *Anal.* ber. für C₁₅H₂₃N: C 82.89, H 10.67, N 6.44. gef.: C 82.80, H 10.62, N 6.33.

## Patentansprüche

1. Verfahren zur Herstellung von 2-Arylethylaminderivaten der Formel (I),
Aryl-CHR¹-CR²(NR³R⁴)-(CHR⁵R⁶)ₙ-CHR⁷R⁸ (I)
worin R¹ bis R⁶
gleich oder verschieden Wasserstoff, C₁-C₂₄-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, Naphthyl, Fluorenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoroalkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, C₁-C₉-Trifluoromethylalkyl, Trifluormethyl, Fluoro, Nitro, Hydroxy, Trifluormethylsulfonato, Thio, Thiolato, C₁-C₈-substituierte Amino der Formen, NH-Alkyl-C₁-C₈, NH-Aryl-C₅-C₆, N-Alkyl₂-C₁-C₈, N-Aryl₂-C₅-C₆, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, C₁-C₇-CO₂H, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃ R⁹₂, CONH₂, CONR⁹₂, CONHR⁹, wobei R⁹ entweder ein C₁-C₈-Alkyl oder C₆-Aryl darstellt, C₁-C₆-Trialkylsilyl, sein können,
wobei auch die einzelnen Substituenten R⁵ bzw. R⁶ in den n Untereinheiten für unterschiedliche Substituenten stehen können, und
worin R⁷ und R⁸
gleich oder verschieden Wasserstoff, C₁-C₂₄-Alkyl, C₃-C₁₂-Oycloalkyl, wobei der Cyclus auch 1-2 Heteroatome ausgewählt aus der Gruppe N, O, S, P enthalten kann und die Ringgröße 3-12 beträgt, Phenyl, Naphthyl, Fluorenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, Fluor, OH, NO₂, CN, O-C₁-C₂₄-Alkyl, O-C₃-C₁₂-Cycloalkyl, wobei der Cyclus auch 1-2 Heteroatome ausgewählt aus der Gruppe N, O, S, P enthalten kann und die Ringgröße 3-12 beträgt,
O-C₆-C₁₄-Aryl, O-C₂-C₁₃-Heteroaryl, wobei die Zahl der Heteroatome ausgewählt aus der Gruppe N,O, S, P 1 bis 4 betragen kann und die Ringgröße 3-14 beträgt,
N-(C₁-C₂₄)₂-Alkyl, N-(C₃-C₁₂)₂-Cycloalkyl, wobei der Cyclus auch 1-2 Heteroatome ausgewählt aus der Gruppe N, O, S, P enthalten kann und die Ringgröße 3-12 beträgt,
N-(C₆-C₁₄-Aryl)₂, N-(C₂-C₁₃-Heteroaryl)₂, wobei die Zahl der Heteroatome ausgewählt aus der Gruppe N, O, S, P 1 bis 4 betragen kann und die Ringgröße 3-14 beträgt,
O₂C-C₁-C₂₄-Alkyl, O₂C-C₃-C₁₂-Cycloalkyl wobei der Cyclus auch 1-2 Heteroatome ausgewählt aus der Gruppe N, O, S, P enthalten kann und die Ringgröße 3-12 beträgt, O₂C-C₆-C₁₄-Aryl,
NR¹⁰-CO-(C₁-C₂₄)₂-Alkyl, NR¹⁰-CO-(C₃-C₁₂)₂-Cycloalkyl, wobei der Cyclus auch 1-2 Heteroatome ausgewählt aus der Gruppe N, O, S, P enthalten kann und die Ringgröße 3-12 beträgt,
NR¹⁰-CO-(C₆-C₁₄-Aryl)₂, NR¹⁰-CO-(C₂-C₁₃-Heteroaryl)₂, wobei die Zahl der Heteroatome ausgewählt aus der Gruppe N, O, S, P enthalten kann und die Ringgröße 3-14 beträgt, wobei R¹⁰ C₁-C₈-Alkyl oder C₆-Aryl darstellt, Si-(C₁-C₂₄)₄-Alkyl, Si-(C₃-C₁₂)₄-Cycloalkyl, wobei der Cyclus auch 1-2 Heteroatome ausgewählt aus der Gruppe N, O, S, P enthalten kann und die Ringgröße 3-12 beträgt,
Si-(C₆-C₁₄-Aryl)₄, Si-(C₂-C₁₃-Heteroaryl)₄, wobei die Zahl der Heteroatome ausgewählt aus der Gruppe N, O, S, P 1 bis 4 betragen kann und die Ringgröße bevorzugt 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoroalkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, C₁-C₉-Trifluoromethylalkyl, Trifluormethyl, Fluoro, Nitro, Hydroxy, Trifluormethylsulfonato, Thio, Thiolato, C₁-C₈-substituierte Amino der Formen, NH-Alkyl-C₁-C₈, NH-Aryl-C₅-C₆, N-Alkyl₂-C₁-C₈, N-Aryl₂-C₅-C₆, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, C₁-C₇-CO₂H, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃R⁹₂, CONH₂, CONR⁹₂, CONHR⁹, wobei R⁹ entweder ein C₁-C₈-Alkyl oder C₆-Aryl darstellt, C₁-C₆-Trialkylsilyl, sein können
und worin Aryl in Formel (I)
C₃-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt,
wobei diese Gruppen selbst jeweils ein- oder mehrfach substituiert sein können und diese Substituenten dabei unabhängig voneinander Wasserstoff, C₁-C₂₀-Alkyl, C₁-C₁₀-Fluoroalkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₂-C₁₃-Heteroaryl, wobei die Zahl der Heteroatome ausgewähit aus der Gruppe N, O, S, P 1 bis 4 betragen kann und die Ringgröße 3-14 beträgt, C₁-C₁₀-Alkoxy, C₆-C₁₄-Aryloxy, C₁-C₉-Trifluoromethylalkyl, Trifluormethyl, Fluoro, Nitro, Hydroxy, Trifluormethylsulfonato, Thio, Thiolato, C₁-C₈-substituierte Amino der Formen NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NHCOH, NH-CO-Alkyl-C₁-C₈, NH-CO-Aryl-C₅-C₆, NHCOO-Alkyl-(C₁-C₄); NHCOO-Aryl-(C₃-C₈); Cyano, C₁-C₆-Acyloxy, SO₂-Aryl-(C₃-C₆), SO-Aryl-(C₃-C₆) SO₂-Alkyl-(C₁-C₆), SO-Alkyl-(C₁-C₆), Sulfinato, Sulfonato der Formen SO₃H und SO₃R⁹, P(Phenyl)₂, CHCHCO₂H, P-Alkyl₂-(C₁-C₈), P-Aryl₂-(C₃-C₈), PO-Phenyl₂, POAlkyl₂-(C₁-C₄), Phosphato der Formen PO₃H₂, PO₃HR⁹ und PO₃ R⁹₂ wobei R⁹, CONH₂, CONR⁹₂, CONHR⁹, entweder C₁-C₈-Alkyl oder C₆-Aryl darstellt, C₁-C₆-Trialkylsilyl, sein können, und
und worin n für eine Zahl zwischen 0 und 11 steht,
durch Umsetzung von aromatischen Olefinen der allgemeinen Formeln (IIa-c)
Aryl-CHR¹-CHR²-(CR⁵R⁶)ₐ-CR⁵=CR⁶-(CR⁵R⁶)_{b}-CHR⁷R⁸ (IIa)
Aryl-CHR¹-CR²=CR⁵-(CR⁵R⁶)_{c}-CHR⁷R⁸ (IIb)
Aryl-CHR¹-CHR²-(CR⁵CR⁶)_{c}-CR⁵=CR⁷CR⁸ (IIc)
mit Aminen der allgemeinen Formel (III),
R³R⁴NH (III)
worin bei den Formeln des Typs II und III R¹ bis R⁸ die zuvor zu den Formeln des Typs (I) angegebena Bedeutung besitzen und a sowie b für eine Zahl zwischen 0 - 9 mit der Bedingung, daß a + b < 10 ist und c für eine Zahl zwischen 0 - 10 steht,
in Gegenwart einer Base.

2. Verfahren nach Anspruch 1 **dadurch gekennzeichnet, daß** die Substituenten R¹ bis R⁶ für Wasserstoff, C₁-C₈-Alkyl, C₆-C₁₄-Aryl, C₃-C₁₂-Cycloalkyl, wobei der Cyclus auch 1-2 Heteroatome ausgewählt aus der Gruppe N, O, S, P enthalten kann und die Ringgröße 3-12 beträgt,
wobei diese Gruppen selbst jeweils ein- bis dreifach substituiert sein können und diese Substituenten dabei unabhängig voneinander Wasserstoff, C₁-C₂₀-Alkyl, C₆-C₁₄-Aryl, C₃-C₁₂-Oycloalkyl, wobei der Cyclus auch 1-2 Heteroatome ausgewählt aus der Gruppe N, O, S, P enthalten kann und die Ringgröße 3-12 beträgt, C₁-C₁₀-Alkoxy, , C₁-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, Fluor, Trifluormethyl, N-Alkyl₂-C₁-C₈, N-Aryl₂-C₅-C₆.

3. Verfahren nach einem der Ansprüche 1 oder 2 **dadurch gekennzeichnet, daß** die Substituenten R¹ bis R⁶ unabhängig voneinanderfür Wasserstoff, C₁-C₈-Alkyl, C₆-C₁₄-Aryl, C₃-C₁₂-Cycloalkyl, wobei der Cyclus auch 1-2 Heteroatome ausgewählt aus der Gruppe N, 0, S, P enthalten kann und die Ringgröße 3-12 beträgt.

4. Verfahren nach einem der Ansprüche 1 bis 3 **dadurch gekennzeichnet, daß** die Substituenten R⁷ und R⁸ unabhängig voneinander Wasserstoff, Fluor, Trifluormethyl, C₆-C₁₄-Aryl, O-C₁-C₈-Alkyl, O-C₃-C₁₂-Cycloalkyl, wobei der Cyclus auch 1-2 Heteroatome ausgewählt aus der Gruppe N, O, S, P enthalten kann und die Ringgröße 3-12 beträgt, O-C₆-C₁₄-Aryl, O-C₂-C₁₃-Heteroaryl, wobei die Zahl der Heteroatome ausgewählt aus der Gruppe N, O, S, P 1 bis 4 betragen kann und die Ringgröße bevorzugt 3-14 beträgt,
N-(C₁-C₈)₂-Alkyl, N-(C₃-C₁₂)₂-Cycloalkyl, wobei der Cyclus auch 1-2 Heteroatome ausgewählt aus der Gruppe N,O, S, P enthalten kann und die Ringgröße 3-12 beträgt,
N-(C₆-C₁₄-Aryl)₂, N-(C₂-C₁₃-Heteroaryl)₂, wobei die Zahl der Heteroatome ausgewählt aus der Gruppe N,O, S, P 1 bis 4 betragen kann und die Ringgröße 3-14 beträgt,
wobei diese Gruppen selbst jeweils ein- bis dreifach substituiert sein können und diese Substituenten dabei unabhängig voneinander Wasserstoff, C₁-C₂₀-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₃-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₆-C₁₄-Aryloxy, C₃-C₁₃-Heteroaryloxy, wobei die Zahl der Heteroatome ausgewählt aus der Gruppe N, O, S, P 1 bis 4 betragen kann und die Ringgröße 3-14 beträgt, N-Alkyl₂-C₁-C₈, N-Aryl₂-C₅-C₆, Trifluormethyl, Fluoro sein können.

5. Verfahren nach einem der Ansprüche 1 bis 4 **dadurch gekennzeichnet, daß** die Substituenten R⁷ und R⁸ unabhängig voneinander Wasserstoff C₃-C₁₄-Aryl, C₂-C₁₃-Heteroaryl, wobei die Zahl der Heteroatome ausgewählt aus der Gruppe N, O, S, P 1 bis 4 betragten kann und die Ringgröße 3-14 beträgt, bedeuten.

6. Verfahren nach einem der Ansprüche 1 bis 5 **dadurch gekennzeichnet, daß** der Arylrest in Formel (I) für C₃-C₁₄-Aryl, C₂-C₁₃-Heteroaryl, wobei die Zahl der Heteroatome ausgewählt aus der Gruppe N, O, S, P 1 bis 4 betragten kann und die Ringgröße 3-14 beträgt, wobei diese Gruppen selbst jeweils ein- bis dreifach substituiert sein können und diese Substituenten dabei unabhängig voneinander Wasserstoff, C₁-C₂₀-Alkyl, C₃-C₁₂-Cycloalkyl, wobei der Cyclus auch 1-2 Heteroatome ausgewählt aus der Gruppe N, O, S, P enthalten kann und die Ringgröße 3-12 beträgt, Phenyl, C₆-C₁₄-Aryl, C₂-C₁₃-Heteroaryl, wobei die Zahl der Heteroatome ausgewählt aus der Gruppe N, O, S, P 1 bis 4 betragen kann und die Ringgröße 3-14 beträgt, C₁-C₁₀-Alkoxy, C₁-C₉-Trifluoromethylalkyl, Trifluormethyl, Fluoro, Nitro, Hydroxy, Trifluormethylsulfonato, Thio, Thiolato steht.

7. Verfahren nach einem der Ansprüche 1 bis 6 **dadurch gekennzeichnet, daß** eine Mischung aus Edukten der Formel (II) eingesetzt wird.

8. Verfahren nach einem der Ansprüche 1 bis 7 **dadurch gekennzeichnet, daß** die eingesetzte Base eine Brönsted-Base ist.

9. Verfahren nach Ansprüche 1 bis 8, **dadurch gekennzeichnet, daß** als Base Alkali- oder/und Erdalkalialkoholate, Alkali- oder/und Erdalkaliamide, Alkali-oder/und Erdalkalielemente oder/und Alkali- oder/und Erdalkalikohlenwasserstoffe oder/und Hydroxide bevorzugt von Lithium, Natrium, Kalium, Calcium, Magnesium, Cäsium oder/und Superbasen, wie alkalimetalldotierte Zeolithe eingesetzt werden.

10. Verfahren nach einem der Ansprüche 1 bis 9, **dadurch gekennzeichnet, daß** man die Base in katalytischen Mengen von 0.01 eq bis 0.5 eq (bezogen auf Amin) verwendet.

11. Verfahren nach einem der Ansprüche 1 bis 10, **dadurch gekennzeichnet, daß** die Reaktion in einem inerten organische Lösungsmittel oder Lösungsmittelgemisch durchgeführt wird.

12. Verfahren nach einem der Ansprüche 1 bis 10, **dadurch gekennzeichnet, daß** kein Lösungsmittel verwendet wird.

13. Verfahren nach einem der vorstehenden Ansprüche, **dadurch gekennzeichnet, daß** die Reaktion bei Temperaturen von -70 bis 200 °C abläuft; vorzugsweise bei 0 bis 180 °C, besonders bevorzugt 20 bis 140 °C.

14. Verfahren nach einem der vorstehenden Ansprüche, **dadurch gekennzeichnet, daß** die Reaktion in Gegenwart eines chelatisierenden Amins als Co-Katalysator durchgeführt wird.
